# FASCICULE DE BREVET EUROPEEN

(11) **EP 4 192 359 B1**
(45) Date de publication et mention de la délivrance du brevet: **30.04.2025**
(21) Numéro de dépôt: 21759109.8
(22) Date de dépôt: 06.08.2021
(51) Int. Cl.: A61B 8/08, A61B 8/12

(54) **COMPLEXE SONDE DE DERIVATION VENTRICULAIRE EXTERNE ET STYLET ECHOGRAPHIQUE POUR UNE POSE SOUS GUIDAGE PAR IMAGERIE CONTINUE**
EXTERNER VENTRIKELKATHETER GEKOPPELT MIT EINEM SONOGRAPHISCHEN MANDRIN ZUR BILDGEFÜHRTEN PLATZIERUNG
EXTERNAL VENTRICULAR DRAINING PROBE COUPLED TO ECHOGRAPHY STYLET FOR POSITIONING UNDER CONTINUOUS GUIDED IMAGING

(30) Priorité: 06.08.2020 FR 2008332
(43) Date de publication de la demande: 14.06.2023
(73) Titulaire: Echopole, 69001 Lyon (FR)
(72) Inventeur: LENFANT, Marc, 21000 Dijon (FR); PORTELLA, Thibault, 21000 Dijon (FR)
(74) Mandataire: IP Trust
(86) Numéro de dépôt international: PCT/FR2021/051452
(87) Numéro de publication internationale: WO 2022/029395

(56) Documents cités:
- WO-A1-96/29011
- US-A1- 2007 083 100
- US-A1- 2014 358 007

## Description

### DOMAINE TECHNIQUE DE L'INVENTION

La présente invention se rapporte à un dispositif chirurgical de dérivation ventriculaire destiné à dériver de façon transitoire (DVE : Dérivation Ventriculaire Externe) ou permanente (par exemple : DVP Dérivation Ventriculo-Péritonéale) le liquide céphalo-rachidien présent dans la boîte crânienne, à l'aide d'un drain implanté dans les ventricules et relié à un système de drainage, lorsque le liquide céphalo-rachidien ne peut circuler librement. L'invention concerne à la fois la sonde de DVE et de DVP, et leur utilisation pendant l'étape de pose, pour le traitement de l'hydrocéphalie aiguë ou chronique et donc de la dilatation ventriculaire. La présente invention trouve ses applications industrielles dans le domaine médical, et notamment dans le domaine chirurgical.

L'hydrocéphalie correspond à une accumulation anormale de liquide au sein du système ventriculaire, augmentant le volume de ce dernier et donc de la pression intracrânienne. Le drainage ventriculaire est effectué avec un cathéter afin de contrôler la pression intracrânienne en dérivant le liquide cérébrospinal (LCS) et de ce fait de diminuer la pression intracrânienne.

La sonde de DVE est constituée d'un cathéter de drainage ventriculaire, généralement en silicone, placé sur un guide rigide que le chirurgien utilise pour implanter la sonde de DVE dans le système ventriculaire du patient. Un petit trou est percé à travers le crâne (trou de trépan) et la sonde de DVE est insérée au travers de la dure-mère puis du cerveau, transfixiant le parenchyme cérébral jusqu'au ventricule cible.

Le placement à main levée de la sonde de DVE nécessite une estimation par le neurochirurgien de la position tridimensionnelle du ventricule cible, généralement sur la base de repères anatomiques externes. Le ventricule ne mesure généralement qu'entre 0,5 et 3 cm de diamètre et peut être situé à une profondeur de vue comprise entre 5 et 50 millimètres, et de préférence de 4 cm ou plus. Une fois la position du ventricule cible estimée, le cathéter de DVE est poussé à travers le cerveau vers le ventricule cible. La méthode à main levée ne fournit aucun moyen de tenir compte des irrégularités potentielles de l'anatomie du patient qui ne sont pas apparentes à l'extérieur. Des facteurs tels que les lésions expansives intracrâniennes (hématome, œdème, tumeur ,...), les cavités porencéphaliques séquellaires, la variabilité génétique, etc., peuvent également affecter la localisation du ventricule cible.

Le placement « intuitif » d'un cathéter oblige parfois le praticien à faire plusieurs tentatives, entraînant plus d'un passage au travers du tissu cérébral pour accomplir le placement requis du cathéter de DVE.

D'après la littérature, jusqu'à 65% des sondes de dérivations ventriculaires posées ont une extrémité distale en position non-optimale (en dehors de la cible ventriculaire optimale). Parmi celles-ci, près d'un tiers sont non fonctionnelles et nécessitent une révision et une réinsertion. Les complications possibles associées au mauvais placement d'un cathéter de DVE peuvent inclure une hémorragie intracérébrale, un accident vasculaire cérébral, des dommages des structures cérébrales adjacentes, ainsi que la nécessité d'une nouvelle opération pour remplacer le cathéter mal positionné. Des taux d'infections plus élevés sont également signalés lorsque plusieurs tentatives de placement de la DVE sont nécessaires.

Plusieurs solutions ont été explorées, notamment l'assistance du positionnement du cathéter avec un système d'imagerie extérieur au crâne (échographie à la surface du parenchyme cérébral en parallèle de la sonde de DVE nécessitant un second trou de trépan, scanner cérébral avec pose en condition stéréotaxique ou guidée par la réalité virtuelle), l'utilisation d'un guide GHAJAR pour contraindre le positionnement par rapport à la surface du crâne, l'utilisation d'un stylet à ultrasons introduit dans le drain pour fournir une information de profondeur par échographie linéaire qui guide l'utilisateur vers le ventricule.

### ÉTAT DE LA TECHNIQUE ANTÉRIEURE

On connaît dans l'état de la technique le brevet WO2015108917 décrivant un dispositif de dérivation implantable pour drainer du liquide cérébro-spinal d'un espace sous-arachnoïdien d'un patient, qui comprend une dérivation ayant des première et seconde extrémités opposées, la seconde extrémité étant conçue pour pénétrer dans une paroi d'un sinus sigmoïde, transversal, droit ou sagittal du patient, un clapet de non-retour, un passage creux s'étendant entre la seconde extrémité et le clapet de non-retour de telle sorte que du liquide cérébro-spinal peut être drainé par la seconde extrémité et expulsé à travers le clapet, et un mécanisme accouplé à la dérivation et conçu pour ancrer la dérivation à un emplacement souhaité près de l'espace sous-arachnoïdien.

On connaît aussi l'article Dual Orientation 16-MHz Single-Element Ultrasound Needle Transducers for Image-Guided Neurosurgical Intervention (auteurs Yun Jiang, Zhen Qiu, et Al.) School of Engineering & Physical Sciences Institute of Sensors, Signals & Systems Institute of Mechanical, Process & Energy Engineering Energy Academy. Cet article concerne l'incorporation d'un appareil à ultrasons dans le type d'aiguilles à biopsie couramment utilisées comme outil interventionnel pour fournir un retour aux neurochirurgiens pendant les interventions chirurgicales. Pour identifier le chemin le plus approprié pour accéder à un site tissulaire ciblé, des transducteurs à élément unique qui orientés vers l'avant ou sur le côté ont été utilisés.

On connaît par ailleurs, dans un domaine différent qui est celui de la ponction rachidienne permettant de prélever ponctuellement un échantillon de liquide céphalo-rachidien non pas dans la boîte crânienne, mais au niveau de la colonne vertébrale, la demande de brevet WO2017192603 qui porte sur un système de placement d'aiguille comprenant une aiguille et un élément transducteur à ultrasons fixé à l'extrémité distale de l'aiguille. Le système comprend également un ensemble de contrainte d'aiguille configuré pour recevoir et contraindre l'aiguille uniquement à des degrés de liberté de rotation dans une plage de mouvement angulaire. Le système comprend également un processeur de données ultrasonores configuré pour communiquer avec l'élément transducteur afin de recevoir des signaux de détection d'ultrasons et communiquer avec le système de capteur d'aiguille pour recevoir des signaux d'orientation angulaire de l'aiguille.

Cette solution de l'art antérieur concerne les interventions pratiquées par des urgentistes ou neurologues pour prélever du liquide céphalo-rachidien (LCR), un liquide vital dans le diagnostic de nombreuses maladies et affections du système nerveux central (SNC). Pour effectuer cette procédure, un médecin palpe le bas du dos et identifie les vertèbres L3 à L5. Une fois identifié, le médecin procède à l'application d'un anesthésique local avant d'insérer et d'avancer une aiguille, généralement une aiguille de Quincke.

Cette solution n'est pas adaptée à une intervention pour implanter un drain dans les ventricules destiné à être relié à un système de drainage, lorsque le liquide céphalo-rachidien ne peut circuler librement.

On connaît aussi la demande de brevet US20070083100A1 décrivant un cathéter de ventriculostomie présentant une paroi de cathéter définissant un passage de lumière centrale, et un dispositif de stylet amovible positionné dans le passage de lumière centrale. Une sonde à ultrasons est disposée dans la paroi du cathéter et reste en place à l'extrémité distale du cathéter lorsque le stylet est retiré. Ainsi, il peut être utilisé pour fournir une imagerie et une surveillance ultrasonores sensiblement continues pendant le positionnement, pendant le drainage de fluide ou pendant l'insertion d'instruments médicaux dans le cerveau via le passage de la lumière centrale du cathéter. L'extrémité distale du cathéter est toujours ouverte, ce qui conduit à la pénétration de fluides corporels.

La demande de brevet WO1996029011A1 décrit un stylet (1) rigide à fibre optique et à ultrasons, placé dans un cathéter intracrânien modifié, en Silastic, permet une visualisation indirecte et directe en temps réel à travers le bout distal du cathéter. Ce stylet est pourvu de fibres optiques ainsi que d'un transducteur à ultrasons permettant une vision à travers l'extrémité du cathéter.

La demande de brevet US2014358007 concerne le positionnement d'un appareil médical à l'intérieur ou à proximité d'un compartiment sensiblement rempli de fluide à l'intérieur d'un corps humain, ledit positionnement étant aidé par ultrason impulsion-écho et une technique d'étirement temporel. Dans un mode de réalisation, un petit transducteur ultrasonore est placé à proximité de la pointe d'un cathéter destiné à être positionné à l'intérieur d'un ventricule cérébral pour drainer le liquide céphalorachidien.

### INCONVÉNIENTS DE L'ART ANTÉRIEUR

Les solutions de l'art antérieur ne sont pas totalement satisfaisantes car elles ne fournissent qu'une information de profondeur selon un mode unidimensionnel n'explorant qu'une seule ligne qui est la ligne de tir axiale de la sonde ultrasonore. Cette information n'aide que partiellement le praticien sur la pertinence du guidage et l'orientation de la sonde. Elles sont conçues pour minimiser la section afin de permettre le passage dans des cathéters de drainage fins de moins de 1,7 mm de section.

Pour exploiter les informations fournies par les solutions de l'art antérieur, le praticien doit apprendre à les interpréter, ce qui peut nécessiter un temps important avant qu'il ne soit en mesure de les interpréter immédiatement, de manière réflexe, lors d'une intervention.

### EXPOSÉ DE L'INVENTION

La présente invention vise à pallier les inconvénients de l'état de la technique par une système de drainage du liquide céphalo-rachidien comprenant une sonde de DVE et un stylet échographique insérable dans ladite sonde de DVE caractérisé en ce que ledit stylet échographique comprenne un tube rigide muni à son extrémité distale d'un transducteur à balayage formant un faisceau dont l'axe est dirigé selon l'axe longitudinal dudit stylet échographique.

Selon son acception la plus générale, l'invention concerne un système de drainage du liquide céphalo-rachidien comprenant une sonde de DVE ou de DVP selon la revendication 1.

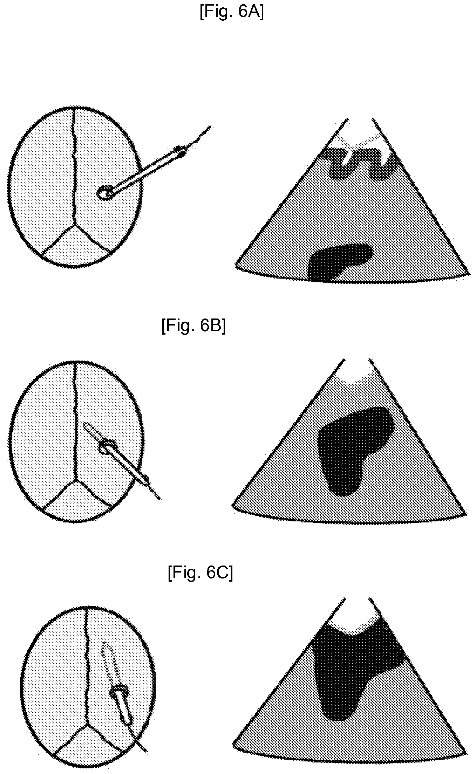

Elle est constituée par un tube souple d'un diamètre extérieur compris entre 1,9 et 5 millimètres et un stylet muni à son extrémité distale d'un transducteur échographique, insérable dans ledit tube, et un échographe comportant un écran de visualisation d'une image calculée en fonction des signaux fournis par ladite sonde échographique, caractérisé en ce que :
- ledit stylet comprend un tube rigide d'un diamètre extérieure correspondant au diamètre intérieur dudit tube contenant seulement les fils d'alimentation dudit transducteur, à l'exclusion d'une fibre optique, ledit tube rigide muni à son extrémité distale d'un transducteur d'un diamètre identique au diamètre dudit tube rigide et présentant une profondeur d'imagerie dans l'axe du tube rigide comprise entre 0 et 100 millimètres,
- l'extrémité distale dudit tube (10) étant fermée par pointe (12) pleine et deformable
- l'extémité proximale dudit tube (figure 6) présente un système de solidarisation avec l'extrémité proximale de la sonde d'échographie. Ce système de solidarisation a 5 objectifs 1) limiter l'impaction longitudinale dudit stylet échographie dans le tube de DV lors de son insertion 2) restreindre la rotation entre le tube (10) et le stylet échographique, 3) fixer la profondeur d'insertion du stylet dans le tube, 4) créer un système solidaire entre le stylet échographique et la dérivation afin d'empécher un mobilité de l'un ou l'autre des deux éléments de l'ensemble et 5) générer un espace liquidien en distalité de la sonde d'échographie propice à son exploitation.
- Le système de solidarisation peut être de type à pas de vis, baïonette, ancre, velcro ou cran d'arrêt cette liste étant non limitative. Dans une variante, l'extrémité proximale du tube porteuse du système de solidarisation est renforcée et détachable de sa portion fonctionnelle. La portion amovible pourra être pré-poinçonnée ou sectionnable en per-opératoire par un matériel tranchant type scalpel ou ciseau médical. Favorablement, cette portion détachable sera transparente afin de pouvoir visuellement confirmer la bonne solidarisation de l'ensemble. Dans une autre variante, la portion de fixation est un pas de vis (type Huer-lock) et est non-amovible, facilitant et sécurisant son raccordement à la tubulure de recueil et sa poche (figure 6) permettant ainsi de limiter les désadaptations par rapport à un système conventionnel d'emboitage.

Dans une variante, le tube présente un ou plusieurs alésage latéraux internes de plus grand axe longitudinal complémentaire à l'alésage central. Certains ou la totalité de ces alésages répondent à la forme complémentaire du stylet échographe (sous forme d'ailettes segmentaires ou de lame longitudinales) afin de maximiser leur solidarisation et de restreinte le degré de rotation en distalité de l'ensemble. Dans une variante, un ou plusieurs de ces alésages permettront avantageusement d'instiller depuis l'extrémité proximale de l'ensemble, un liquide trans-échogène tel que du sérum physiologique à l'interface des éléments piézo-éléctrique du stylet échographe et de la face interne de la pointe du tube de DV.

La section dudit tube est de forme ronde. Dans une variante, la section du tube sera asymétrique, épaissie en regard des alésages internes latéraux afin de conserver une résistance satisfaisante aux courbures après retrait du stylet échographe (figure 6) Le stylet échographe et éventuellement le tube de DV présentent un marqueur visuel proximal permettant de connaitre l'orientation de la sonde d'échographie et faciliter le repérage spatial de l'opérateur durant l'insertion intra-parenchymateuse de l'ensemble.

L'échographe fournit une imagerie échographique de mode B.

Selon une variante, ledit échographe fournit une imagerie échographique de mode B associé à au moins une autre modalité d'imagerie comprenant le mode couleur, le doppler pulsé, la puissance et le mode 3D.

Avantageusement, ledit transducteur est un transducteur à balayage.

Selon une variante, le système comporte en outre une enveloppe stérile entourant ledit stylet et dont l'extrémité dans laquelle est engagée ledit transducteur contient un gel d'échographie.

Selon une autre variante, le fond distal du tube présente une forme complémentaire de la forme extérieure dudit transducteur.

Selon un premier mode de réalisation, ladite pointe présente une forme conique. Selon un autre mode de réalisation, ladite pointe présente une forme hémisphérique/alternative ayant pour objectif de limiter la génération d'aberrations optiques par la déviation du faisceaux d'ultrasons qu'elle engendre (responsable in fine d'artefacts d'échographie).

La pointe et/ou l'ensemble du tube de DVE sont constitué de matériaux biocompatibles avec des propriétés biomécaniques similaires à celles habituellement attendue pour une sonde de dérivation et, avantageusement, avec un matériau présentant le plus faible coefficient d'atténuation du faisceau d'ultrason possible. L'épaisseur de cette pointe pourra avantageusement être réduite dans le même objectif.

Avantageusement, ladite pointe contient un gel échographique ou de l'eau stérile instillée. Cette instillation pourra se faire avant l'assemblage échographe-tube par immersion préalable du tube de DV dans une bassine de sérum physiologique ou par son instillation via la lumière interne du tube. Dans une variante, l'ensemble sera fixé et l'installation antérograde se fera par les alésages internes latéraux, ou par ponction direct à l'aide d'une aiguille de la pointe conique du tube de DV.

Selon une variante, ledit système comprend en outre un calculateur exécutant un programme informatique de prétraitement du signal fourni par ledit transducteur à balayage, ledit prétraitement consistant à appliquer une compensation des variations des conditions de propagation dans ladite pointe du tube.

De préférence la fréquence de travail dudit transducteur est comprise entre 4 Mhz et 15 Mhz, plus avantageusement de 10 Mhz.

Selon une variante la pointe de la sonde comporte un marqueur échogène

### BRÈVE DESCRIPTION DES FIGURES

D'autres caractéristiques et avantages de l'invention ressortiront à la lecture qui suit d'exemples de réalisation détaillés, en référence aux figures annexées qui représentent respectivement :

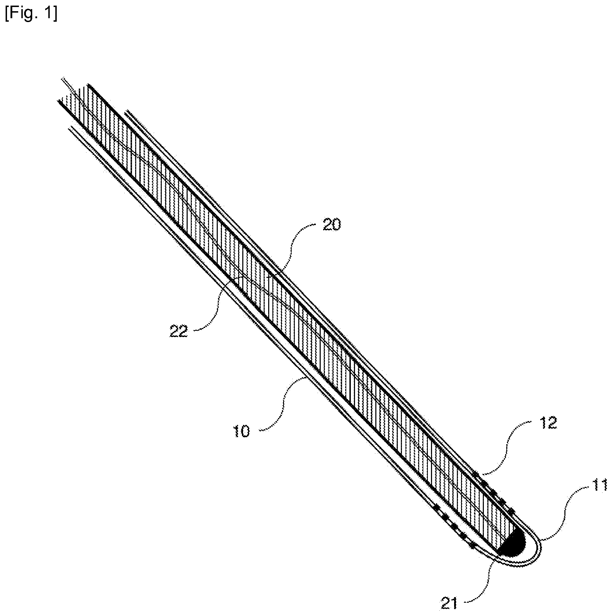

[Fig. 1] la figure 1 représente une vue en coupe d'un système selon l'invention.
[Fig. 2] la figure 2 représente une vue de l'extrémité de la sonde selon un premier exemple de réalisation.
[Fig. 3] la figure 3 représente une vue de l'extrémité de la sonde selon un deuxième exemple de réalisation.
[Fig. 4] la figure 4 représente une vue en coupe de l'extrémité de la sonde selon un troisième exemple de réalisation.
[Fig. 5] la figure 5 représente une vue en coupe de l'extrémité de la sonde selon un quatrième exemple de réalisation
[Fig. 6A] la figure 6A représente une vue du stylet selon une première position
[Fig. 6B] la figure 6B représente une vue du stylet selon une deuxième position
[Fig. 6C] la figure 6C représente une vue du stylet selon une troisième position
[Fig. 7A] la figure 7A représente une vue en coupe d'une première variante de la géométrie du tube selon l'invention
[Fig. 7B] la figure 7B représente une vue en coupe d'une deuxième variante de la géométrie du tube selon l'invention
[Fig. 7C] la figure 7C représente une vue en coupe d'une troisième variante de la géométrie du tube selon l'invention
[Fig. 7D] la figure 7D représente une vue en coupe d'une quatrième variante de la géométrie du tube selon l'invention
[Fig. 7E] la figure 7E représente une vue en coupe transversale d'une première variante du tube selon l'invention
[Fig. 7F] la figure 7F représente une vue en coupe transversale d'une première variante du tube selon l'invention
[Fig. 7G] la figure 7G représente une vue en coupe transversale d'une deuxième variante du tube selon l'invention
[Fig. 8A] la figure 8A représente une vue en coupe du tube selon l'invention avec une pointe pleine
[Fig. 8B] la figure 8B représente une vue en coupe du tube selon l'invention avec une pointe pleine après introduction du liquide transéchogène
[Fig. 8C] la figure 8C représente une vue en coupe du tube selon l'invention avec une pointe pleine après introduction du liquide et du stylet échographique
[Fig. 8D] la figure 8D représente une vue en coupe du tube selon l'invention avec une pointe creuse
[Fig. 8E] la figure 8E représente une vue en coupe du tube selon l'invention avec une pointe creuse après introduction du liquide transéchogène
[Fig. 8F] la figure 8F représente une vue en coupe du tube selon l'invention avec une pointe creuse après introduction du stylet échographique puis introduction du liquide par la rainure
[Fig. 8G] la figure 8G représente une vue en coupe du tube selon l'invention avec une pointe creuse après introduction du liquide et du stylet échographique
[Fig. 9] la figure 9 représente une vue en coupe du tube selon l'invention
[Fig. 10] la figure 10 représente une vue en coupe du stylet échographique
[Fig. 11] la figure 11 représente une vue en coupe du système formé par le stylet engagé dans le tube de dérivation ventriculaire.

### DESCRIPTION D'UN EXEMPLE DE RÉALISATION

L'invention concerne un système composé d'une sonde de DVE ou de DVP constituant un tube de drainage (10) et d'un stylet échographe pour implantation guidée (20).

Le diamètre extérieur du tube de drainage (10) est typiquement compris entre 1,9 millimètres et 5 millimètres, et le diamètre intérieur de 1,7 millimètres à 3,5 millimètres. Le diamètre extérieur du stylet (20) est typiquement inférieur de 0,5 millimètre par rapport au diamètre interne du tube de drainage, avec un canal médian pour le passage des fils électrique reliant le transducteur à l'échographe.

L'épaisseur de la pointe à l'extrémité distale dudit tube est comprise entre 1 et 7 mm selon la puissance de la sonde d'échographie et la forme de la pointe. Cette pointe est pleine, ou plus favorablement, elle présente un alésage central ellipsoïdal, ou quadrangulaire épousant la surface du stylet doppler. Dans un assemblage cet alésage est maximal, de forme ellipsoïdal, conique, pyramidal ou quadrangulaire voir d'une forme proche, afin d'accueillir le complexe stylet doppler-enveloppe souple. Il peut s'agir par exemple de silicone, d'un matériau à base de silicone, c'est-à-dire comprenant aux moins 10% de silicone, caoutchouc, plastique, polyamide, polyéther bloc amide, polycarbonate, polyimide, polytétrafluoroéthylène, cette liste étant non limitative.

Le tube de drainage (10) est perforé distalement et latéralement par de multiples trous (12) d'environ 2 mm de diamètre sur une étendue environ 20 mm. Ces ouvertures peuvent être des pores, des trous, des orifices, des fentes et traversent de part en part la membrane du tube (10). Ces ouvertures sont de préférence d'une taille adaptée au passage de l'eau, du liquide céphalorachidien et du sang. Lesdits pores, trous, orifices ou fentes pourront être adaptés selon leur capacité à permettre l'écoulement du liquide cérébro-spinal, avec des formes variables pouvant être cylindrique, conique ou ovale, cette liste est non limitative. La distance entre les trous est variable, pouvant aller de 1 à 4 mm pour permettre le bon écoulement du liquide cérébro-spinal.

L'extrémité forme une pointe conique ou pyramidale ou ellipsoïdale (11) et est non rigide. Cette pointe est pleine avec un alésage interne proximal afin d'épouser la forme de l'extrémité distale de la sonde d'échographie, mais peut présenter un alésage interne de forme adaptée à recevoir un complexe enveloppe souple-stylet doppler ou une interface liquidienne.

Le tube de drainage souple (10) est destiné à être implanté pour une courte ou longue durée sur un patient afin de drainer le système ventriculaire en cas d'hydrocéphalie aiguë ou chronique. Ce tube (10) est raccordé à un système de dérivation par gravité à l'aide d'un sac de recueil une fois qu'il est mis en place en cas de DVE et à un système de drainage relié au péritoine ou au système vasculaire en cas de DVP.

En cas de DVP, le tube de drainage souple présente les mêmes caractéristiques dans sa portion distale (pointe, trous, pores ou orifices de drainage). La longueur intracrânienne est de 4 à 9 cm avec un angle droit permettant l'application du tube à la boite crânienne à l'aide d'un réservoir, poursuivi par un tube de drainage qui sera relié à la valve de dérivation. La zone d'union entre la dérivation et le stylet échographique pourra être sectionner afin d'être relier à la valve.

Le canal de ce tube de drainage (10) permet d'accueillir un stylet échographe (20) formé par un mandrin rigide amovible dont la longueur dépasse celle du tube de drainage (10), pour faciliter l'introduction du dispositif dans le parenchyme cérébral et faciliter son retrait après pose de ladite sonde de DVE/DVP. Ce stylet échographique comprend un transducteur ultrasonore à balayage (21) alimenté par des fils (22). Il s'agit par exemple d'un transducteur de marque ALOKA UST 534. Ce stylet échographique s'insère dans le tube de drainage le long de la zone d'alésage à l'aide de l'ailette et sa position finale se verrouille par le système de fixation proximal afin de solidariser l'ensemble et de générer un espace liquidien distal propice à l'exploitation de la sonde d'échographie. L'introduction de l'ensemble se fera orientée selon le marqueur du stylet échographe indiquant l'orientation du faisceau échographique et de sa partie antérieure.

L'élément actif du transducteur est constitué principalement d'un matériau piézoélectrique, éventuellement piézocomposite, éventuellement multicouche, et d'un ensemble d'au moins deux électrodes qui permettent de créer un champ électrique dans l'épaisseur du matériau piézoélectrique. De préférence, une ou plusieurs couches d'adaptation acoustique sont intégrées dans cet élément actif, sur la face avant de l'élément actif, pour faciliter le transfert acoustique vers l'avant du transducteur.

Le transducteur est avantageusement collé à l'extrémité distale du stylet (20) par une colle époxy.

Ce transducteur peut être constitué d'un réseau de micro-transducteurs piézo-électriques, ou d'un transducteur unique animé d'un mouvement oscillant par un support de type MEMS. Il délivre un signal 2D ou 3D de l'environnement en avant de la pointe conique (11) afin de permettre à l'opérateur de guider l'avancement et l'orientation du mandrin rigide lors de la mise en place du drain au travers du parenchyme cérébral d'un patient. Les signaux sont exploités par un échographe 2D en mode B pour fournir une image de la zone en avant de la sonde.

La fréquence de travail est typiquement de 10 Mhz. Le transducteur (21) est relié à un échographe.

Ce dispositif échographique doit bénéficier d'un mode échographique
« B » (imagerie anatomique 2D), éventuellement accompagnée d'un mode « C » (couleur), d'un mode « D » (doppler), d'un mode puissance et/ou d'un mode 3D, avec une profondeur d'imagerie comprise entre 0 et 50-100 millimètres. La sonde d'échographie est préférentiellement de type linéaire, curvilinéaire, ou micro-convexe mais peu correspondre à tout type de sonde d'échographie permettant d'obtenir une image échographie de type B selon l'axe longitudinal dudit stylet (microsonde type IVUS (intravasculaire) adaptée, sonde trapézoïdale, ...).

Le signal fournit par le transducteur peut être corrigé, en cas d'inhomogénéité de la pointe (11) par un calculateur appliquant un traitement numérique de compensation des variations de transmission induite par le matériau constitutif de la pointe (11) et des variations d'épaisseur en fonction de l'angle du tir.

### CONFIGURATION DE LA POINTE

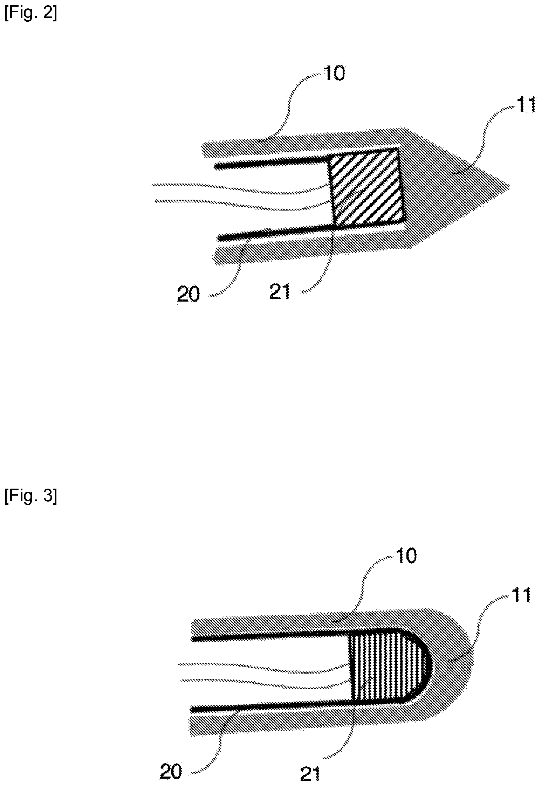

Les figures 2 à 5 illustrent différentes configurations de la pointe (11) du tube souple (20). Elle peut être conique comme illustré par la figure 2 ou hémisphérique comme illustré par la figure 3.

L'extrémité distale de la dérivation ventriculaire est produite de manière à être la plus atraumatique possible, dans un matériau biocompatible le plus transéchogène possible.

Son épaisseur et sa conformation géométrique auront pour but de réduire les artefacts d'aberration optique et l'atténuation du faisceau d'ultrasons.

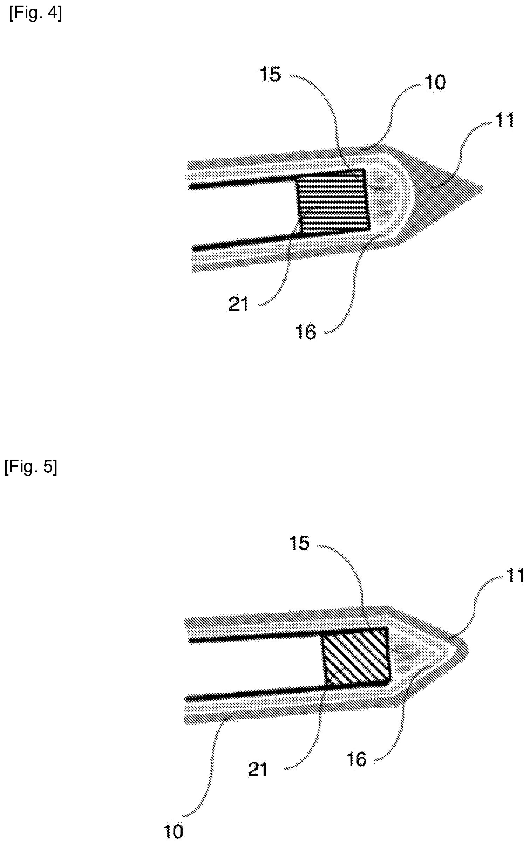

Elle peut aussi recevoir un gel échographique (15) logé entre la sonde (21) et l'intérieur de la pointe (11) comme représenté en figure 4 ou 5.

Le gel d'échographie stérile est solidarisé à la pointe du stylet échographique par une enveloppe souple stérile (16), réalisant un complexe enveloppe souple-stylet doppler qui est chargé au sein de la sonde de dérivation. Dans cette variante, le diamètre interne de la sonde de DVE est discrètement supérieur au diamètre externe dudit complexe enveloppe souple-stylet doppler (Fig.2). La longueur de l'enveloppe souple est telle qu'une fois déroulé sur le stylet doppler, elle dépasse en tout temps la surface de la boite crânienne et reste inférieure à la longueur du stylet doppler (environ entre 6 et 20cm).

Alternativement, un liquide trans-echogène tel que du sérum physiologique est directement instillé à l'interface transducteur-pointe du tube de dérivation. Cette instillation pouvant se réaliser avant l'assemblage du complexe tube-stylet échographe, ou après l'assemblage par le biais d'un alésage interne du tube complémentaire à l'alésage central recevant ledit stylet ou par ponction direct à l'aide d'une fine aiguille d'un espace à la face postérieur de la pointe dudit tube. Ainsi, dans ces variantes, la longueur de la portion du stylet d'échographie à l'intérieur du tube de dérivation sera discrètement inférieure à l'alésage interne dudit tube afin de produire cet espace liquidien propice à l'utilisation de la sonde d'échographie. Optionnellement, la pointe de la sonde comporte un marqueur échogène latéral dont le but est de former une référence visible sur l'image échographique transcranienne.

### CONFIGURATION DE LA PARTIE PROXIMALE

Les figures 6 illustrent le pas de vis mâle et femelle entre la sonde d'échographie et le tube de dérivation permettant d'obtenir une solidarisation entre les deux composants. Le pas de vis une fois verrouillé permet 1) de limiter l'impaction longitudinale du tube de DV lors de son insertion, 2) de restreindre la rotation entre le tube (10) et le stylet échographique, 3) de fixer la profondeur d'insertion du stylet dans le tube, 4) créer un système solidaire entre le stylet échographique et la dérivation afin d'empêcher un mobilité de l'un ou l'autre des deux éléments de l'ensemble et 5) générer un espace liquidien en distalité de la sonde d'échographie propice à son exploitation. Ce dernier point assure d'avoir un espace liquidien étanche à l'interface tube/échographie quelques soit les mouvements imprimés à l'ensemble à l'extrémité distale de l'ensemble (figure 6).

Dans une variante, le pas de vis dudit tube de dérivation permet son raccordement à la tubulure de receuil et à la poche (X) limitant les risques de désadaptation par rapport à un système d'emboitage conventionnel. Dans une autre variante, le système de fixation est présent sur le segment le plus proximal du tube et est amovible en per-opératoire.

### CONFIGURATION DE LA PARTIE ANTERIEUR DU TUBE

Les figures 6A à 6C illustrent la partie antérieure du dit tube de forme ellipsoïdale avec une partie antérieure plus épaisse avec une rainure à sa partie interne permettant de recevoir l'ailette de ladite sonde d'échographie afin d'éviter les faux trajets et de sécuriser l'introduction.

A sa partie antérieure externe se trouve un marquage indiquant l'orientation de la sonde d'échographie et du sens d'introduction au sein du parenchyme cérébral (figure 6).

### MISE EN ŒUVRE DE L'INVENTION

Une ouverture ponctiforme de la peau est réalisée à l'aide de repère anatomique en avant de la suture coronale à 2 ou 3 cm latéralement de la ligne médiane. Ensuite, un trou de trépan d'environ 5 mm à 3 cm de diamètre est réalisé, avec ouverture et parfois coagulation de la dure-mère.

Instillation d'eau stérile avec une seringue le long de l'alésage interne de la dérivation afin de produire une collection liquidienne distale propice à l'usage de la sonde d'échographie.

Le stylet échographe (20) est engagé dans l'alésage interne central du tube (10) de la sonde de DV pour la rigidifier, en insérant l'ailette de la sonde d'échographie dans l'alésage interne latéral de la dérivation ventriculaire pour éviter tout faux trajet jusqu'au verrouillage par le système de fixation proximal. L'assemblage final retrouve le transducteur échographique enchâssé dans la pointe du tube de dérivation ventriculaire avec une interface liquidienne garanti par l'instillation de sérum physiologique préalable. L'ensemble permettant d'acquérir des informations sur l'environnement de la pointe du tube (10) et orienter les gestes de l'opérateur. Le verrouillage du pas de vis se fait avant d'insérer l'ensemble au contact du parenchyme cérébral et se fait par contrôle visuel des marqueurs à la surface du stylet échographe et du tube, ainsi que par contrôle visuel direct dans une variante. L'opérateur pose la pointe de la sonde de DV à la surface du parenchyme cérébral et visualise la cible ventriculaire. Habituellement, elle correspond à la corne frontale d'un ventricule latéral homolatéral dans le cadre d'une DVE, et dans le carrefour ventriculaire homolatéral pour les DVP. Une fois repérée, l'opérateur enfonce progressivement le complexe à travers le parenchyme cérébral, sous guidage échographique continu jusqu'à atteindre sa cible.

Le stylet échographique (20) est ensuite retiré du tube (10), en dévissant le pas de vis proximal et en faisant coulisser l'ailette dans l'alésage interne de la dérivation, libérant la lumière du drain de DVE et autorisant un reflux immédiat de LCS par son extrémité proximale. Le mécanisme aspiratif peut se baser uniquement sur la gravité. Pour ce faire, la poche de collection du liquide doit être située en contrebas de la tête afin d'obtenir un effet siphon.

A l'extrémité opposée à l'extrémité perforée (12), un système de succion muni d'un embout de raccordement à une tubulure (type Micro Vac, Elite Surgical Supplies) est vissé dans la sonde de DVE, afin de permettre un raccordement sécurisé diminuant le risque de désadaptation et diminuant le risque infectieux inhérent à cette complication. La dépression engendrée est adaptée au contexte clinique, généralement de 1 bar. Dans le cas d'une DVP, le système de drainage est relié à une valve de drainage régulant le débit et le tout est poursuivi par le cathéter abdominal se trouvant soit dans le péritoine soit dans le système vasculaire au niveau de la veine cave.

Le volume de LCS qui est ainsi extrait de la boite crânienne permet de contrôler et de traiter l'hypertension intracrânienne sévère, évitant ainsi les multiples complications qu'elle induit.

En cas de réalisation d'une dérivation ventriculo-péritonéale (DVP) ou atriale (DVA), l'incision cutanée est réalisée en rétro-auriculaire, un trou de trépan de 2 à 3 cm est réalisé. On coagule la dure-mère puis on l'incise. Le stylet échographe est chargé dans la sonde de DVP. L'opérateur pose la pointe du complexe stylet échographique inséré dans la sonde de DVP à la surface du parenchyme cérébral et visualise la cible ventriculaire. Habituellement, elle correspond au carrefour ventriculaire. Une fois repérée, l'opérateur enfonce progressivement le complexe à travers le parenchyme cérébral, sous guidage échographique continu jusqu'à atteindre sa cible. Le stylet échographique est ensuite retiré, libérant la lumière du cathéter de DVP et autorisant un reflux immédiat de LCS par son extrémité proximale. L'ensemble sera relié à une valve de dérivation et à un cathéter abdominal ou atrial.

### DIAMÈTRE EXTÉRIEUR DU TUBE

Pour des applications de drainage sans saignement intraventriculaire, on choisira de préférence un tube présentant un diamètre extérieur de 1,5 à 1,9 millimètres.

Pour des applications de drainage avec saignement intraventriculaire, on choisira de préférence un tube présentant un diamètre extérieur de 2,5 à 5 millimètres, et de préférence inférieur à 4 millimètres.

### CONFIGURATION DE LA POINTE DU TUBE

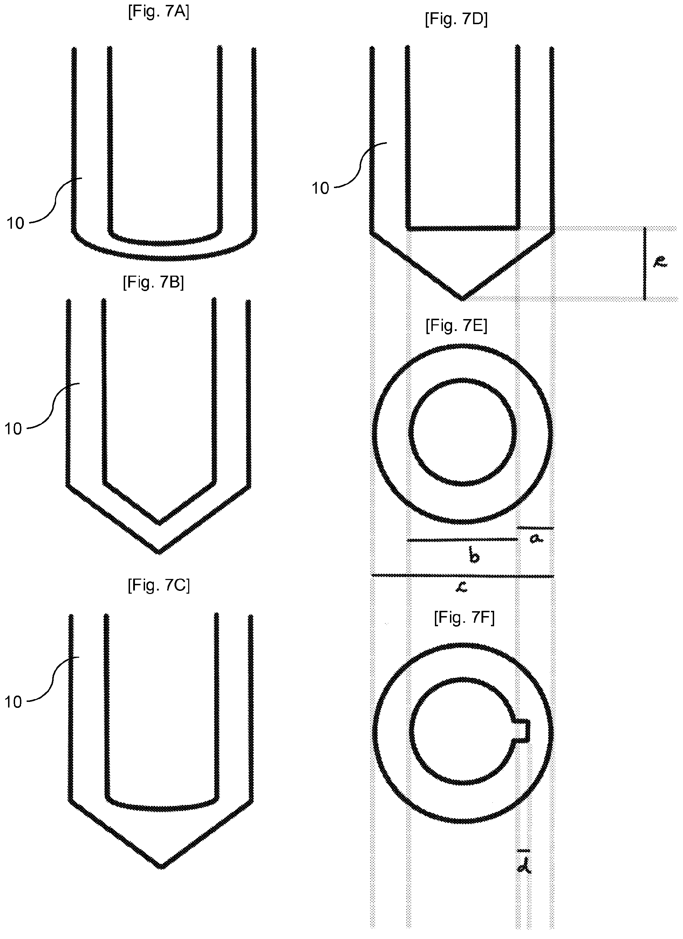

Les figures 7A à 7G illustrent différentes réalisations de la pointe déformable du tube (10). Elle peut présenter :
- Une extrémité légèrement concave avec une peau frontale d'épaisseur sensiblement constante (figure 7A) pour former une extrémité frontale creuse bombée
- Une extrémité conique avec une peau frontale d'épaisseur sensiblement constante (figure 7B) pour former une extrémité pointue creuse
- Une extrémité conique avec un fond transversal plan (figure 7C) pour former une extrémité pointue plein

La figure 7D illustre la hauteur a de la pointe pleine et déformable, comprise entre 0,7 mm et 7 mm.

L'épaisseur n de la paroi du tube (10) est d'environ 0,7 mm et l'alésage intérieur est compris entre 1,9 et 3 mm (figure 7E).

Le diamètre extérieur du tube (10) est compris entre 1,9 et 4 mm.

La figure 7F illustre une variante ou la paroi du tube (10) présente une rainure (17) formant un alésage d'une profondeur inférieure à l'épaisseur de la paroi, soit inférieur à 0,7 mm. Éventuellement, l'épaisseur de la paroi est augmentée au niveau de la rainure.

Cet alésage permet d'injecter dans le fond du tube (10) un liquide transéchogène.

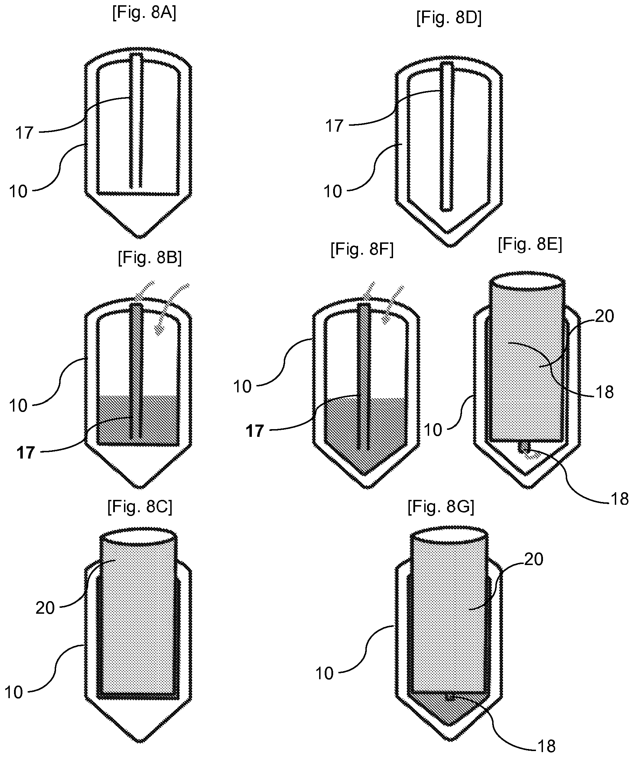

Les figures 8A à 8C illustrent le tube (10) avec une pointe pleine et déformable, et optionnellement une rainure pour injecter un liquide transéchogène avant l'introduction du stylet portant à son extrémité distale un transducteur ultrasonore. Ce liquide peut être introduit via l'alésage latéral (17) ou via l'alésage central.

Les figure 8D à 8G illustrent le tube (10) avec une pointe creuse et déformable, et optionnellement (figure 7G) une rainure (17) pour injecter un liquide transéchogène sous le stylet portant à son extrémité distale un transducteur ultrasonore, la circulation s'effectuant par la lumière interne (18) (figure 8E).

### Instillation du liquide transéchogène

Selon un premier mode d'utilisation, on introduit le liquide dans le tube avant l'insertion du stylet échographe, de sorte à ce que la sonde vienne en contact avec le liquide remplissant le volume intérieur de la pointe creuse.

Selon un deuxième mode d'utilisation, le stylet échographe est introduit dans le tube, et ensuite on injecte du liquide dans le tube par le biais d'un alésage longitudinal (17) formé dans la paroi du tube (10).

Selon une troisième variante, le stylet échographe est introduit dans le tube et ensuite on injecte du liquide transéchogène dans le volume intérieur de la pointe de la sonde par l'intermédiaire d'une seringue dont l'aiguille traverse la paroi du tube (10).

### Système de dérivation ventriculaire

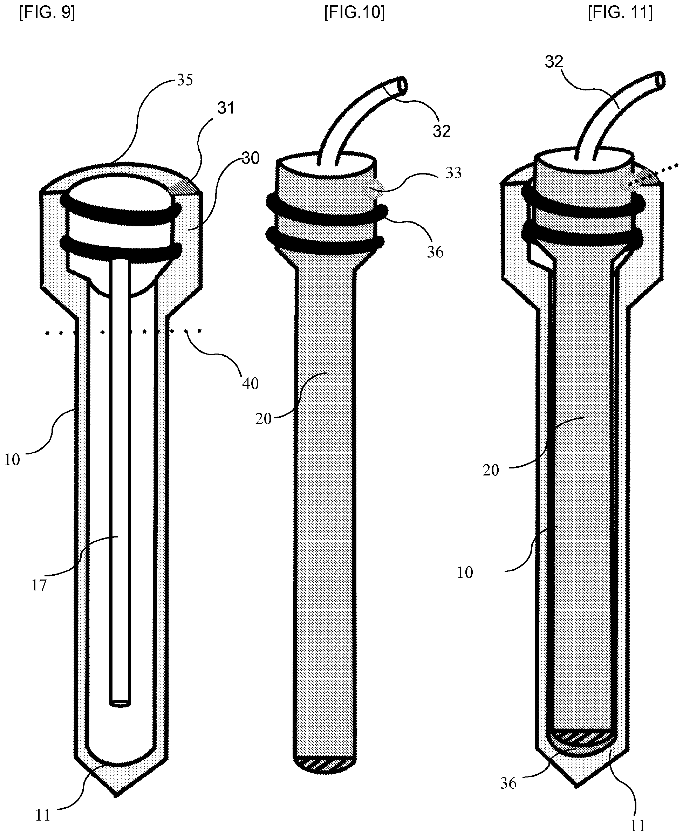

Les figures 9 à 11 illustrent l'invention et présente la sonde de dérivation ventriculaire seule (figure 9), le stylet échographique seul (figure 10) et le stylet engagé dans la sonde de dérivation ventriculaire (figure 11).

La sonde (10) présente un tronçon (30) avec une paroi plus épaisse du côté de l'ouverture d'introduction du stylet (20) et/ou réalisé en un matériau plus rigide que le matériau de la sonde (10). Le bord de l'extrémité ouverte de la sonde présente un marqueur (31) visuel permettant de disposer d'un référentiel angulaire. Le tronçon (30) présente un filetage intérieur (35) pour la fixation du stylet (22) présentant un filetage (36) complémentaire. Ce filetage (35) permet aussi de fixer ensuite une poche de drainage de liquide.

La sonde (10) présente une rainure d'affaiblissement (40) permettant optionnellement de sectionner la partie amont de la sonde (10).

Le stylet (22) présente également un marqueur visuel (33) permettant de vérifier l'orientation angulaire du stylet par rapport à la sonde (10) et son bon alignement angulaire, ainsi que la direction d'observation.

Le fond de la pointe (11) contient du liquide transéchogène (36) assurant l'interface entre la sonde échographique et la sonde de dérivation (10). Le stylet (20) dépasse légèrement du tronçon épaissi (30) du tube de dérivation (10).

Selon une variante, l'épaississement pariétal est asymétrique.

## Revendications

1. - Système de drainage du liquide céphalo-rachidien pour la dérivation ventriculaire comprenant une sonde de DVE ou de DVP constituée par un tube (10) souple et un stylet (20) muni à son extrémité distale d'un transducteur échographique (21), insérable dans ledit tube (10), et un échographe comportant un écran de visualisation d'une image anatomique calculée en mode B en fonction des signaux fournis par ledit transducteur échographique (21),
- ledit stylet (20) comprend un tube rigide d'un diamètre extérieur correspondant au diamètre intérieur dudit tube (10) contenant seulement les fils d'alimentation dudit transducteur échographique (21), à l'exclusion d'une fibre optique, ledit tube rigide muni à son extrémité distale d'un transducteur (21) d'un diamètre identique au diamètre dudit tube (10) et présentant une profondeur d'imagerie dans l'axe du tube (10), ledit stylet comportant un système de solidarisation proximal du complexe,
**caractérisé en ce que** l'extrémité distale dudit tube (10) est fermée par un pointe (12) creuse fermée et déformable formant une poche de réception d'un liquide transéchogène.

2. - Système de drainage du liquide céphalo-rachidien pour la dérivation ventriculaire selon la revendication 1 **caractérisé en ce que** ledit tube (10) présente un filetage (36) pour l'engagement temporaire d'un stylet d'échographie.

3. - Système de drainage du liquide céphalo-rachidien pour la dérivation ventriculaire selon la revendication 1 **caractérisé en ce que** ledit échographe fournit une imagerie échographique de mode B associé à au moins une autre modalité d'imagerie comprenant le mode couleur, le doppler pulsé, la puissance et le mode 3D.

4. - Système de drainage du liquide céphalo-rachidien pour la dérivation ventriculaire selon la revendication 1 **caractérisé en ce que** ledit transducteur est un transducteur à balayage.

5. - Système de drainage du liquide céphalo-rachidien pour la dérivation ventriculaire selon la revendication 1 **caractérisé en ce qu'**il comporte en outre une enveloppe stérile entourant ledit stylet (20) et dont l'extrémité dans laquelle est engagée ledit transducteur (21) contient un gel d'échographie.

6. - Système de drainage du liquide céphalo-rachidien pour la dérivation ventriculaire selon la revendication 1 **caractérisé en ce que** le fond distal du tube (10) présente une forme complémentaire de la forme extérieure dudit transducteur (21).

7. - Système de drainage du liquide céphalo-rachidien pour la dérivation ventriculaire selon la revendication 1 **caractérisé en ce que** ladite pointe présente une forme conique.

8. - Système de drainage du liquide céphalo-rachidien pour la dérivation ventriculaire selon la revendication 1 **caractérisé en ce que** ladite pointe présente une forme hémisphérique.

9. - Système de drainage du liquide céphalo-rachidien pour la dérivation ventriculaire selon la revendication 1 **caractérisé en ce qu'**il comprend en outre un calculateur exécutant un programme informatique de prétraitement du signal fourni par ledit transducteur à balayage, ledit prétraitement consistant à appliquer une compensation des variations des conditions de propagation dans ladite pointe (12) du tube (10).

10. - Système de drainage du liquide céphalo-rachidien pour la dérivation ventriculaire selon la revendication 1 **caractérisé en ce que** la pointe de la sonde comporte un marqueur échogène.

11. - Système de drainage du liquide céphalo-rachidien pour la dérivation ventriculaire selon la revendication 1 **caractérisé en ce qu'**un système de solidarisation permette d'unir l'ensemble des deux éléments et de produire un espace liquidien stable à l'extrémité distale de ladite sonde d'échographie.

12. - Système de drainage du liquide céphalo-rachidien pour la dérivation ventriculaire selon la revendication 1 **caractérisé en ce que** la partie interne du dit tube de dérivation présente un alésage optimal pour un écoulement liquidien afin de créer un milieu propice à l'utilisation de ladite sonde d'échographie à sa partie interne distale.

13. - Système de drainage du liquide céphalo-rachidien pour la dérivation ventriculaire selon la revendication 1 **caractérisé en ce que** l'extrémité proximale du dit tube de dérivation comporte un connecteur permettant d'unir ledit tube de dérivation et le système de drainage composé d'un tube et d'un sac de recueil.

## Patentansprüche

1. Zerebrospinalflüssigkeitsdrainagesystem für die ventrikuläre Ableitung, umfassend eine DVE- oder DVP-Sonde, die aus einem flexiblen Schlauch (10) und einem Stilett (20) gebildet ist, das an seinem distalen Ende mit einem Ultraschallwandler (21) versehen ist, das in den Schlauch (10) einführbar ist, und ein Ultraschallgerät, das einen Bildschirm zum Anzeigen eines anatomischen Bildes aufweist, das in einem B-Modus in Abhängigkeit der Signale berechnet wird, die durch den Ultraschallwandler (21) bereitgestellt werden,
- wobei das Stilett (20) einen starren Schlauch mit einem Außendurchmesser umfasst, der dem Innendurchmesser des Schlauchs (10) entspricht, der nur die Stromversorgungsdrähte des Ultraschallwandlers (21) enthält, ausschließlich einer optischen Faser, wobei der starre Schlauch an seinem distalen Ende mit einem Wandler (21) mit einem Durchmesser versehen ist, der identisch zu dem Durchmesser des Schlauchs (10) ist und eine Bildgebungstiefe in der Achse des Schlauchs (10) vorweist, wobei das Stilett ein proximales Befestigungssystem des Komplexes aufweist,
**dadurch gekennzeichnet, dass** das distale Ende des Schlauchs (10) durch eine verschlossene und verformbare hohle Spitze (12) verschlossen ist, die eine Tasche zum Aufnehmen einer transechogenen Flüssigkeit ausbildet.

2. Zerebrospinalflüssigkeitsdrainagesystem für die ventrikuläre Ableitung nach Anspruch 1, **dadurch gekennzeichnet, dass** der Schlauch (10) ein Gewinde (36) zum vorübergehenden Eingreifen eines Ultraschallstiletts vorweist.

3. Zerebrospinalflüssigkeitsdrainagesystem für die ventrikuläre Ableitung nach Anspruch 1, **dadurch gekennzeichnet, dass** das Ultraschallsystem eine B-Modus-Ultraschallbildgebung bereitstellt, die mit mindestens einer weiteren Bildgebungsmodalität verknüpft ist, umfassend den Farbmodus, den gepulsten Doppler-, den Leistungs- und den 3D-Modus.

4. Zerebrospinalflüssigkeitsdrainagesystem für die ventrikuläre Ableitung nach Anspruch 1, **dadurch gekennzeichnet, dass** der Wandler ein Abtastwandler ist.

5. Zerebrospinalflüssigkeitsdrainagesystem für die ventrikuläre Ableitung nach Anspruch 1, **dadurch gekennzeichnet, dass** es ferner eine sterile Hülle aufweist, die das Stilett (20) umgibt und deren Ende, in dem der Wandler (21) in Eingriff steht, ein Ultraschallgel enthält.

6. Zerebrospinalflüssigkeitsdrainagesystem für die ventrikuläre Ableitung nach Anspruch 1, **dadurch gekennzeichnet, dass** der distale Boden des Schlauchs (10) eine Form vorweist, die komplementär zu der Außenform des Wandlers (21) ist.

7. Zerebrospinalflüssigkeitsdrainagesystem für die ventrikuläre Ableitung nach Anspruch 1, **dadurch gekennzeichnet, dass** die Spitze eine konische Form vorweist.

8. Zerebrospinalflüssigkeitsdrainagesystem für die ventrikuläre Ableitung nach Anspruch 1, **dadurch gekennzeichnet, dass** die Spitze eine halbkugelförmige Form vorweist.

9. Zerebrospinalflüssigkeitsdrainagesystem für die ventrikuläre Ableitung nach Anspruch 1, **dadurch gekennzeichnet, dass** es ferner einen Computer umfasst, der ein Computerprogramm für eine Vorverarbeitung des Signals ausführt, das durch den Abtastwandler bereitgestellt wird, wobei die Vorverarbeitung darin besteht, eine Kompensation für Variationen von Ausbreitungsbedingungen in der Spitze (12) des Schlauchs (10) vorzunehmen.

10. Zerebrospinalflüssigkeitsdrainagesystem für die ventrikuläre Ableitung nach Anspruch 1, **dadurch gekennzeichnet, dass** die Spitze der Sonde eine echogene Markierung aufweist.

11. Zerebrospinalflüssigkeitsdrainagesystem für die ventrikuläre Ableitung nach Anspruch 1, **dadurch gekennzeichnet, dass** ein Verbindungssystem es ermöglicht, die Anordnung der zwei Elemente zu vereinen und an dem distalen Ende der Ultraschallsonde einen stabilen Flüssigkeitsraum zu erzeugen.

12. Zerebrospinalflüssigkeitsdrainagesystem für die ventrikuläre Ableitung nach Anspruch 1, **dadurch gekennzeichnet, dass** der innere Teil des Ableitungsschlauchs eine optimale Bohrung für einen Flüssigkeitsfluss vorweist, um eine Umgebung zu schaffen, die für die Verwendung der Ultraschallsonde an ihrem distalen inneren Teil geeignet ist.

13. Zerebrospinalflüssigkeitsdrainagesystem für die ventrikuläre Ableitung nach Anspruch 1, **dadurch gekennzeichnet, dass** das proximale Ende des Ableitungsschlauchs einen Verbinder aufweist, der es ermöglicht, den Ableitungsschlauch mit dem Drainagesystem zu vereinen, das aus einem Schlauch und einem Auffangbeutel besteht.

## Claims

1. Cerebrospinal fluid drainage system for ventricular drainage, comprising an EVD or VPS probe consisting of a flexible tube (10) and a stylus (20) provided at its distal end with an ultrasound transducer (21), which can be inserted into the tube (10), and an echograph comprising a screen for displaying an anatomical image calculated in B-mode as a function of the signals supplied by the ultrasound transducer (21),
- the stylus (20) comprises a rigid tube with an outside diameter corresponding to the inside diameter of the tube (10), the rigid tube containing only the supply wires of the ultrasound transducer (21), excluding an optical fiber, the rigid tube provided at its distal end with a transducer (21) of a diameter identical to the diameter of the tube (10) and having an imaging depth in the axis of the tube (10), the stylus comprising a proximal securing system of the complex,
**characterized in that** the distal end of the tube (10) is closed by a closed, deformable hollow tip (12) forming a pocket for receiving a transechogenic liquid.

2. Cerebrospinal fluid drainage system for ventricular drainage according to claim 1, **characterized in that** the tube (10) has a thread (36) for the temporary engagement of an ultrasound stylus.

3. Cerebrospinal fluid drainage system for ventricular drainage according to claim 1, **characterized in that** the echograph provides B-mode ultrasound imaging associated with at least one other imaging modality comprising color mode, pulsed Doppler, power and 3D mode.

4. Cerebrospinal fluid drainage system for ventricular drainage according to claim 1, **characterized in that** the transducer is a scanning transducer.

5. Cerebrospinal fluid drainage system for ventricular drainage according to claim 1, **characterized in that** it further comprises a sterile enclosure surrounding the stylus (20), the end of which, in which the transducer (21) is engaged, contains an ultrasound gel.

6. Cerebrospinal fluid drainage system for ventricular drainage according to claim 1, **characterized in that** the distal bottom of the tube (10) has a shape complementary to the outer shape of the transducer (21).

7. Cerebrospinal fluid drainage system for ventricular drainage according to claim 1, **characterized in that** the tip has a conical shape.

8. Cerebrospinal fluid drainage system for ventricular drainage according to claim 1, **characterized in that** the tip has a hemispherical shape.

9. Cerebrospinal fluid drainage system for ventricular drainage according to claim 1, **characterized in that** it further comprises a computer executing a computer program for preprocessing the signal supplied by the scanning transducer, the preprocessing consisting in applying compensation for variations in the propagation conditions in the tip (12) of the tube (10).

10. Cerebrospinal fluid drainage system for ventricular drainage according to claim 1, **characterized in that** the tip of the probe comprises an echogenic marker.

11. Cerebrospinal fluid drainage system for ventricular drainage according to claim 1, **characterized in that** a securing system makes it possible to unite the assembly of the two elements and to produce a stable fluid space at the distal end of the ultrasound probe.

12. Cerebrospinal fluid drainage system for ventricular drainage according to claim 1, **characterized in that** the internal part of the drain tube has an optimum bore for fluid flow in order to create an environment that is conducive to the use of the ultrasound probe at its distal internal part.

13. Cerebrospinal fluid drainage system for ventricular drainage according to claim 1, **characterized in that** the proximal end of the drain tube comprises a connector for joining the drain tube and the drainage system consisting of a tube and a collection bag.
